# EUROPÄISCHE PATENTSCHRIFT

(11) **EP 1 296 896 B1**
(45) Veröffentlichungstag und Bekanntmachung des Hinweises auf die Patenterteilung: **01.09.2004**
(21) Anmeldenummer: 01951420.7
(22) Anmeldetag: 26.06.2001
(51) Int. Cl.: C02F 11/04, C12M 1/107, C02F 3/20, C02F 3/28

(54) **VORRICHTUNG ZUR DREIPHASENTRENNUNG BEI DER BEHANDLUNG VON ABWASSER UND SCHLAMM**
THREE-PHASE SEPARATION DEVICE FOR TREATING WASTE WATER AND SLUDGE
DISPOSITIF DE SEPARATION A TROIS PHASES POUR LE TRAITEMENT DES EAUX RESIDUAIRES ET DES BOUES

(30) Priorität: 30.06.2000 DE 10031093
(43) Veröffentlichungstag der Anmeldung: 02.04.2003
(73) Patentinhaber: Verink, Johan, 30625 Hannover (DE)
(72) Erfinder: Verink, Johan, 30625 Hannover (DE)
(74) Vertreter: Scheffler, Jörg
(86) Internationale Anmeldenummer: PCT/DE2001/002298
(87) Internationale Veröffentlichungsnummer: WO 2002/002467

(56) Entgegenhaltungen:
- EP-A- 0 096 825
- EP-A- 0 808 805
- CH-A- 648 594
- DE-A- 3 904 326

## Beschreibung

Die Erfindung betrifft eine Vorrichtung zur Dreiphasentrennung bei der Behandlung von Abwasser und Schlamm mit einem in einem Absetzbecken angeordneten Gassammler zum Auffangen des bei dem Umwandlungsprozeß in der Vorrichtung zur Dreiphasentrennung entstehenden oder in diese eingeleiteten Gases, wobei der Gassammler eine das Abgleiten von Klärschlamm ermöglichende und gegenüber der Horizontalen geneigte Außenfläche aufweist.

Eine solche Vorrichtung zur Dreiphasentrennung wird dazu genutzt, um die bei dem aeroben oder anaeroben Umwandlungsprozeß anfallenden Faulgase oder die bei der aeroben Behandlung zugeführten Gase in dem Gassammler aufzufangen. Diese stehen dann im erstgenannten Fall als Energiequelle einer weiteren Nutzung zum Betrieb der Vorrichtung oder auch für sonstige Zwecke oder im zweitgenannten Fall, beispielsweise zu Geruchsentfernung, zur Verfügung.

Aus der Praxis ist es hierzu bereits bekannt, den Gassammler derart winkelförmig auszuführen, daß die beiden Schenkel die geneigte Außenfläche bilden und zugleich ein Volumen einschließen, in welches das Gas von unten ungehindert einströmen kann. Eine ähnliche Vorrichtung ist z.B. Gegenstand der EP-A-0808805 bzw. der EP-A-0096825. Auf der geneigten Außenfläche des Gassammlers gleitet der von oben eingefüllte Klärschlamm im wesentlichen ungehindert ab und setzt sich auf dem Beckengrund ab. Das bei dem nachfolgenden Umwandlungsprozeß entstehende nutzbare Gas steigt anschließend nach oben und gelangt dabei in den Gassammler. Einander benachbarte Gassammler weisen dabei zweckmäßigerweise eine geringfügige Überlappung auf, durch die ein Verlust von Gas, welches anderenfalls durch den Zwischenraum nach oben ungehindert zur Wasseroberfläche aufsteigen könnte, verhindert wird. Der absinkende Klärschlamm gleitet dabei gegebenenfalls auch von einer Außenfläche auf die Außenfläche, wobei der Abstand eines benachbarten Gassammlers derart bemessen sein muß, daß ein Verstopfen des Zwischenraumes verhindert wird. Mehrere Gassammler können nebeneinander und in mehreren Ebenen übereinander angeordnet werden, um so die Leistungsfähigkeit der Vorrichtung zur Dreiphasentrennung zu steigern. Als praxisgerecht hat sich dabei erwiesen, einen Neigungswinkel zwischen 50° und 70° gegenüber der Horizontalen vorzusehen, wodurch gleichermaßen ein zuverlässiges Abgleiten des eingefüllten Klärschlammes und auch ein großflächiges und damit problemloses Auffangen des aufsteigenden Gases realisierbar ist.

Üblicherweise wird der Gassammler aus vorgefertigten Bauelementen oder aus Beton oder Kunststoff vor Ort zusammengesetzt, wodurch eine beliebige Erweiterung in der Ebene sowie auch in weiteren Ebenen übereinander ermöglicht wird.

Als nachteilig hat es sich beim Betrieb der genannten Vorrichtung zur Dreiphasentrennung erwiesen, daß die Konsistenz des erzeugten Klärschlammes oftmals stark schwankt. Infolgedessen kann der Klärschlamm an der Außenfläche des Gassammlers anhaften oder anlagern. Um dem entgegenzutreten ist eine vorhergehende Untersuchung und gegebenenfalls Behandlung des Klärschlammes erforderlich, was mit vergleichsweise hohem Aufwand verbunden ist. Ist es dennoch zu einer Verstopfung der Zwischenräume gekommen, so wird eine aufwendige manuelle Reinigung erforderlich.

Aufgabe der vorliegenden Erfindung ist es daher, eine Vorrichtung zur Dreiphasentrennung der eingangs beschriebenen Art derart auszuführen, daß dadurch in einfacher Weise ein Anhaften von Klärschlamm auf der Außenfläche des Gassammlers sowie eine dadurch gegebenenfalls verursachte Verstopfung der Zwischenräume vermieden werden können. Dabei soll insbesondere eine vorhergehende Behandlung des Klärschlammes entbehrlich sein.

Diese Aufgabe wird erfindungsgemäß durch eine Vorrichtung zur Dreiphasentrennung bei der Behandlung von Abwasser und Schlamm gemäß den Merkmalen des Patentanspruchs 1 gelöst. Die Unteransprüche betreffen besonders zweckmäßige Weiterbildungen der Erfindung.

Erfindungsgemäß ist also eine Vorrichtung zur Dreiphasentrennung vorgesehen, bei welcher die Außenfläche einen zur Anpassung an verschiedene Klärschlämme einstellbaren Neigungswinkel aufweist. Hierdurch wird es möglich, in einfacher Weise eine schnelle Anpassung an unterschiedliche Klärschlämme zu erreichen. Das unerwünschte Anhaften des Klärschlammes, wodurch unter Umständen eine Anhäufung in den Zwischenräumen der benachbarten Gassammler verursacht wird, ist dadurch zuverlässig ausgeschlossen. Hierzu ist die Außenfläche beispielsweise durch ein Gelenk beweglich angeordnet, wobei bereits anhaftender Klärschlamm durch eine vorübergehende Einstellung eines großen Neigungswinkels abgelöst werden kann. Daher können Anlagerungen von Schlamm minimiert oder mühelos entfernt werden, wobei der Neigungswinkel sowohl manuell als auch elektromotorisch verstellbar sein kann. Daher ist eine vorhergehende Bestimmung der charakteristischen Eigenschaften des Klärschlammes nicht erforderlich, so daß der für die erfolgreiche Behandlung des Abwassers erforderliche Aufwand verringert werden kann.
Eine besonders vorteilhafte Ausführungsform der Erfindung ist auch dadurch gegeben, daß der Gassammler zwei in ihrem Neigungswinkel unabhängig voneinander einstellbare, einander zugeneigte Außenflächen aufweist. Hierdurch wird eine problemlose Anpassung an unterschiedliche Eigenschaften der verschiedenen Klärschlämme ebenso berücksichtigt, wie eine räumlich unterschiedliche Verteilung des Klärschlammes in dem Absetzbecken, was gegebenenfalls einen abschnittsweise abweichenden Neigungswinkel erfordert.

Eine andere besonders vorteilhafte Weiterbildung der Erfindung wird auch dadurch erreicht, daß durch den eingestellten Neigungswinkel der Außenfläche zugleich auch das zur Aufnahme des Gases jeweils verfügbare Volumen des Gassammlers bestimmt ist. Hierdurch führt eine Veränderung des Neigungswinkels der Außenfläche zugleich auch zu einer veränderten Kapazität des Gassammlers, so daß zugleich eine einfache Anpassung erreicht wird. Dabei führt ein großer Neigungswinkel unmittelbar zu einem großen Gasvolumen, welches daher problemlos aufgefangen werden kann. Der jeweilige Neigungswinkel eines mit zwei einander zugeneigten Außenflächen versehenen Gassammlers kann dabei sowohl übereinstimmend als auch unabhängig voneinander eingestellt werden.

Besonders vorteilhaft ist auch eine Weiterbildung der vorliegenden Erfindung, bei der mehrere benachbarte Gassammler durch einen gemeinsamen Antrieb in ihrem Neigungswinkel verstellbar sind. Hierdurch wird eine einfache Bewegung der Außenfläche durch eine einheitliche Verstellung der benachbarten Gassammler erreicht. Eine gegenseitige Behinderung benachbarter Gassammler durch einen unterschiedlichen Neigungswinkel und ein dadurch verursachtes ungleichmäßiges Absinken ist daher ausgeschlossen. Die Außenflächen der verschiedenen Gassammler können dabei zu Reparaturzwecken durch eine Entkopplung auch unterschiedlich voneinander beweglich sein.

Das unerwünschte Anhaften von Schlamm wird besonders effektiv dadurch vermieden, daß der Neigungswinkel durch einen Antrieb kontinuierlich veränderbar ist. Durch die beispielsweise mittels eines Exzenterantriebes erzeugte stetige Bewegung läßt sich zudem auch bereits anhaftender Schlamm mühelos entfernen.

Eine andere vorteilhafte Ausgestaltung der vorliegenden Erfindung wird auch dadurch geschaffen, daß die Außenfläche einen flexiblen Abschnitt aufweist. Hierdurch kann eine einfache Verstellung des Neigungswinkels durch eine elastische Verformung der Außenfläche erreicht werden. Weiterhin ist es dadurch möglich, durch eine in schneller Folge wechselnde Verformung, gegebenenfalls auf der Außenfläche anhaftende Partikel des Klärschlammes zu lösen und eine bereits aufgetretene Anhaftung zu entfernen. Dabei können beispielsweise auch Wölbungen einstellbar sein, die das Abgleiten des Klärschlammes verbessern.

Hierzu ist es auch besonders zweckmäßig, wenn die Außenfläche eine gasdichte Folie oder ein Gewebe aufweist. Hierdurch wird eine beliebige Verformbarkeit der Außenfläche realisierbar, die in Abhängigkeit der wesentlichen Parameter des Klärschlammes einstellbar ist. Die gasdichte Folie bzw. das gasdichte Gewebe können hierzu durch eine geeignete Halterung unter Spannung gesetzt werden oder aber auch zunächst frei herabhängen und erst durch den beginnenden Füllvorgang ihre vorbestimmte Form erhalten.

Eine andere vorteilhafte Ausgestaltung der vorliegenden Erfindung wird dadurch geschaffen, daß der Gassammler ein für die Außenfläche bestimmtes Spannmittel aufweist. Hierdurch kann eine auf die Beschaffenheit des einzufüllenden Klärschlammes abgestimmte Spannung der Außenfläche eingestellt werden, durch die einerseits die Formgebung, andererseits auch die Oberflächenbeschaffenheit der Außenfläche bestimmt werden kann. Die dadurch ebenfalls erreichbare Dehnung der Außenfläche verhindert dabei wirkungsvoll ein mögliches Anhaften des Klärschlammes.

Dabei ist es auch besonders günstig, wenn das Spannmittel ein Federelement ist. Es läßt sich an der Außenfläche eine konstante Spannung einstellen, die durch eine geeignete Anordnung des Federelementes von einem gegebenenfalls anhaftenden Klärschlamm derart beeinflußt wird, daß die Spannung geändert und der Klärschlamm dadurch abgelöst wird. Das Federelement kann hierzu beispielsweise als eine Zugfeder ausgeführt sein und an einem freien Ende der Außenfläche angreifen oder aber auch als Druckfeder gegen einen rückwärtigen Bereich der Außenfläche anliegen, um so die erforderliche Spannung auf die Außenfläche zu übertragen.

In besonders einfacher Weise läßt sich die Spannung auch dadurch einstellen, daß das Spannmittel einen Auftriebskörper aufweist. Dabei wird die in dem Absetzbecken auftretende Auftriebskraft genutzt, um die erforderliche Spannung für die Außenfläche aufzubringen. Hierdurch kann insbesondere auch eine einfache Positionierung des Gassammlers durch einen Ausgleich des hierzu einstellbaren Auftriebes und der durch das Eigengewicht verursachten Abtriebskräfte erreicht werden.

Dabei ist es auch besonders günstig, wenn der Auftriebskörper aus Bambus hergestellt ist. Dieser ermöglicht durch seine eingeschlossenen Luftkammem eine einfache Anpassung der gewünschten Auftriebskräfte, wobei die Luftkammern auch zur Reduzierung des Auftriebes durchbrochen werden können. Weiterhin können die Zwischenwände der Luftkammern entfernt und dadurch eine einfache Leitung geschaffen werden. Weiterhin ist Bambus gerade in den für die wirtschaftliche Verwertung wichtigen Ländern nahezu unbegrenzt verfügbar.

Eine besonders vorteilhafte Abwandlung der vorliegenden Erfindung wird auch dadurch erreicht, daß der Gassammler eine an mehreren Umlenkflächen gehaltene, bewegliche Fläche aufweist, mit einem ersten, die Außenfläche bildenden Abschnitt und mit einem zweiten, zur Aufnahme des entstehenden Gases zurückgesetzten Abschnitt. Hierdurch wird in einfacher Weise das Bauelement zugleich außenseitig als Außenfläche und innenseitig als Behälter genutzt. Dadurch reduziert sich der Herstellungsaufwand auf wenige Bauteile, die hierzu problemlos aus Formteilen montiert werden können.

Hierzu ist es auch besonders günstig, wenn die flexible Fläche aus einem Schlauch besteht. Dieser läßt sich problemlos durch einzelne im Inneren des Schlauches angeordnete Umlenkflächen zu der geneigten Außenfläche formen, wobei das zum Auffangen des Gases bestimmte Volumen durch eine weitere, von außen gegen den Schlauch anliegende Umlenkfläche ausgeformt ist. Eine hinsichtlich der Haltbarkeit ungünstige Einfassung der Randabschnitte der Außenfläche entfällt dabei, indem lediglich eine einfach darzustellende Umlenkfläche erforderlich ist. Zugleich wird dadurch eine räumliche Trennung des aufliegenden Klärschlammes von dem aufgefangenen Gas durch einen isolierenden Zwischenraum geschaffen, die hierzu gegen jeweils unterschiedliche Abschnitte des Schlauchumfanges anliegen, wobei eine gegebenenfalls auftretende Undichtigkeit zunächst nicht unmittelbar zu einem Gasverlust führt. Dabei ist es ferner auch denkbar, daß der Schlauch an den Umlenkflächen umlaufend antreibbar ist, um so beispielsweise die Reinigung zu vereinfachen.

Der Gassammler könnte durch Spannseile, die teilweise im Inneren des Schlauches verlaufen, die gewünschte Formgebung erhalten. Besonders vorteilhaft ist hingegen auch eine weitere günstige Ausführungsform der vorliegenden Erfindung, bei der die Umlenkflächen an zumindest einer seitlichen formstabilen Aufnahme fixiert sind. Hierdurch wird die Handhabung des Gassammlers vereinfacht, indem dieser eine von weiteren Bauelementen der Vorrichtung zur Dreiphasentrennung unabhängige Beschaffenheit erhält und dadurch eine nachträgliche Änderung der Positionierung gestattet.

Hierbei ist es auch vorteilhaft, wenn die Vorrichtung mehrere zueinander benachbarte Gassammler aufweist, die einen zueinander einstellbaren Abstand aufweisen. Hierdurch kann der zwischen den benachbarten Gassammlern verbleibende Spalt problemlos verändert werden, um so eine Verstopfung zu vermeiden. Der Abstand kann dabei sowohl- gemeinsam mit dem Neigungswinkel einstellbar sein, wobei insbesondere ein großer Neigungswinkel einen breiten Spalt erfordert, oder auch unabhängig von diesem veränderlich sein.

Eine andere vorteilhafte Abwandlung der Erfindung wird dadurch geschaffen, daß die Außenfläche transmissiv ist. Hierdurch wird eine Begünstigung des Umwandlungsprozesses erreicht, indem das Umgebungslicht, insbesondere das Sonnenlicht, im wesentlichen ungehindert durch den Gassammler hindurchtreten kann und dadurch auch den Grund des Absetzbeckens erreicht. Hierdurch kann die Qualität der dadurch erreichbaren Gasmenge und damit die Wirtschaftlichkeit der Anlage wesentlich verbessert werden.

Besonders vorteilhaft ist es auch, wenn der Gassammler ein Verbindungsmittel zur Positionierung bezüglich eines Druckgasspeichers der Vorrichtung zur Dreiphasentrennung aufweist. Hierbei wird in dem Gassammler das Gas mit lediglich geringem Überdruck gegenüber der Umgebung aufgefangen und anschließend an den Druckgasspeicher weitergeleitet. Der Druckgasspeicher kann hierzu mittels des Verbindungsmittels in einer geeigneten Position zu dem Gassammler fixiert werden, wobei die Weiterleitung des Gases von dem Gassammler zu dem Druckgasspeicher zugleich durch das Verbindungsmittel erfolgen kann. Die zur Vorrichtung zur Dreiphasentrennung gehörenden Bauelemente können auf diese Weise modular aufgebaut und problemlos miteinander verbunden werden, wobei eine Erweiterung ohne weiteres realisierbar ist.

Die Erfindung läßt verschiedene Ausführungsformen zu. Zur weiteren Verdeutlichung ihres Grundprinzips ist eine davon in der Zeichnung dargestellt und wird nachfolgend beschrieben. Diese zeigt in
- Fig. 1: eine perspektivische Ansicht eines Gassammlers;
- Fig. 2: eine Seitenansicht des in Figur 1 gezeigten Gassammlers;
- Fig. 3: eine Seitenansicht eines weiteren Gassammlers.

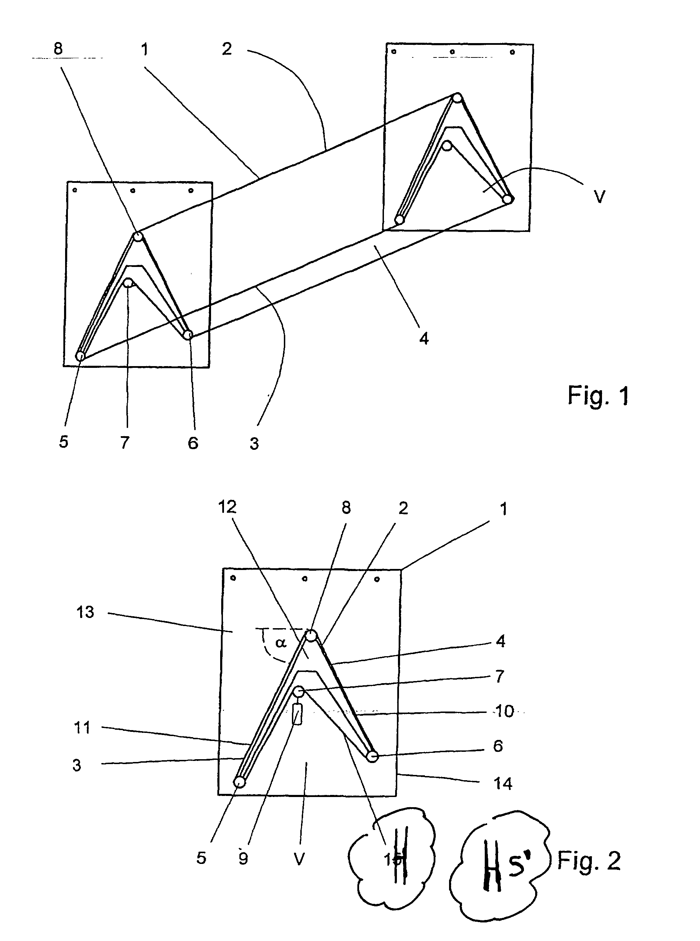

Figur 1 zeigt einen Gassammler 1 einer nicht weiter dargestellten, erfindungsgemäßen Anlage zur Behandlung von Abwasser und Schlamm in einer perspektivischen Ansicht. Zu erkennen ist die im wesentlichen abgewinkelte Ausführung des Gassammlers 1, dessen Schenkel außenseitig jeweils eine Außenfläche 2, 3 bilden und innenseitig ein Volumen V zum Auffangen des Gases begrenzen. Diese Form wird in einfacher Weise durch einen aus einer Folie bestehenden Schlauch 4 geschaffen, der an mehreren Umlenkflächen 5, 6, 7, 8 straff gespannt ist und so die gewünschte Formgebung erhält. Die Umlenkflächen 5, 6, 7, 8 sind dabei in unterschiedlichen Positionen fixierbar, um so eine abweichende Form des Gassammlers 1 einstellen zu können.

Diese abweichende Formgebung wird anhand der Figur 2 weiter erläutert, die den Gassammler 1 in einer Seitenansicht zeigt. Dargestellt sind die Umlenkflächen 5, 6, 7, 8, durch die der Schlauch 4 seine Ausformung erhält. Aufgrund der einfachen Verstellbarkeit der Umlenkflächen 5, 6, 7, 8 läßt sich so insbesondere ein auf den jeweiligen Klärschlamm abgestimmter Neigungswinkel α einstellen, um so ein Anhaften des einzufüllenden Klärschlammes zuverlässig ausschließen zu können. Sollte es aufgrund einer Unachtsamkeit dennoch zu einem Anhaften kommen, so kann der Neigungswinkel α in schneller Folge geändert werden, um so die anhaftenden Partikel "abzuschütteln". Zugleich wird durch den veränderten Neigungswinkel α auch das durch den Gassammler 1 eingeschlossene Volumen V bestimmt, welches zum Auffangen des von unten aufsteigenden Gases zur Verfügung steht. Der Schlauch 4 wird dabei mittels eines als Federelement ausgeführten Spannmittels 9 vorgespannt, so daß die Einhaltung der vorbestimmten Formgebung gewährleistet ist. Der Schlauch 4 weist weiterhin zwischen zwei die Außenfläche 2, 3 bildenden Abschnitten 10, 11 und einem zweiten, das Volumen V begrenzenden Abschnitt 15' eine Verstärkung 12 auf, durch welche die Formstabilität erhöht wird. Außenseitig hat der Gassammler 1 ein mit einer Halteplatte 13 ausgestattetes Verbindungsmittel 14, durch welches einerseits die Umlenkflächen 5, 6, 7, 8 fixiert sind, andererseits zugleich auch der Anschluß an einen nicht dargestellten Druckgasspeicher der Vorrichtung zur Dreiphasentrennung geschaffen wird.

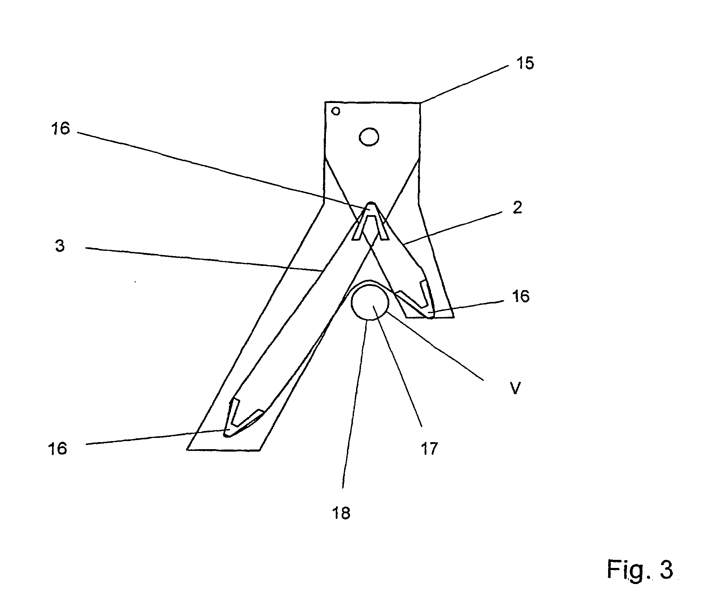

Figur 3 zeigt einen weiteren Gassammler 15 in einer Seitenansicht. Dieser unterscheidet sich von dem in Figur 2 gezeigten Gassammler 1 insbesondere durch eine abweichende Gestaltung der Umlenkflächen 16, die hierbei durch ein Profil erreicht werden und dadurch eine erhöhte Stabilität bei zugleich geringem Gewicht aufweisen. Weiterhin hat der Gassammler 15 ein mit einem Auftriebskörper 17 ausgestattetes Spannmittel 18. Hierdurch stellt sich die erforderliche Spannung der Außenflächen 2, 3 selbsttätig ein, ohne daß hierzu ein zusätzlicher manueller Eingriff erforderlich ist. Der Auftriebskörper 17 kann dabei insbesondere aus Bambus hergestellt werden, so daß eine einfache und kostengünstige Anpassung des erforderlichen Auftriebes, beispielsweise auch durch eine Durchbrechung einzelner Luftkammem, erreicht werden kann. Hierdurch läßt sich in einfacher Weise das gewünschte Gleichgewicht zwischen der Gewichtskraft des Gassammlers 15 und den dieser Gewichtskraft entgegenwirkenden Auftriebskraft erreichen, wobei der Auftrieb des Auftriebskörpers 17 durch das Volumen V des eingeschlossenen Gases zusätzlich erhöht wird. Es können außerdem auch zusätzliche Kontragewichte zur Erzielung des Gleichgewichtes verwendet werden, die hierzu ihrerseits auch zu Feinabstimmung zumindest abschnittsweise mit Flüssigkeit befüllbar sein können.

## Patentansprüche

1. Vorrichtung zur Dreiphasentrennung bei der Behandlung von Abwasser und Schlamm mit einem in einem Absetzbecken angeordneten Gassammler (1, 15) zum Auffangen des bei dem Umwandlungsprozeß in der Vorrichtung zur Dreiphasentrennung entstehenden oder in diese eingeleiteten Gases, wobei der Gassammler (1, 15) eine das Abgleiten von Klärschlamm ermöglichende und gegenüber der Horizontalen geneigte Außenfläche (2, 3) aufweist, **dadurch gekennzeichnet, daß** die Außenfläche (2, 3) einen zur Anpassung an verschiedene Klärschlämme einstellbaren Neigungswinkel (α) aufweist.

2. Vorrichtung zur Dreiphasentrennung nach Anspruch 1, **dadurch gekennzeichnet, daß** der Gassammler (1, 15) zwei in ihrem Neigungswinkel (α) unabhängig voneinander einstellbare, einander zugeneigte Außenflächen (2, 3) aufweist.

3. Vorrichtung zur Dreiphasentrennung nach den Ansprüchen 1 oder 2, **dadurch gekennzeichnet, daß** durch den eingestellten Neigungswinkel (α) der Außenfläche (2, 3) zugleich auch das zur Aufnahme des Gases jeweils verfügbare Volumen (V) des Gassammlers (1, 15) bestimmt ist.

4. Vorrichtung zur Dreiphasentrennung nach zumindest einem der vorhergehenden Ansprüche, **dadurch gekennzeichnet, daß** mehrere benachbarte Gassammler (1, 15) durch einen gemeinsamen Antrieb in ihrem Neigungswinkel (α) verstellbar sind.

5. Vorrichtung zur Dreiphasentrennung nach zumindest einem der vorhergehenden Ansprüche, **dadurch gekennzeichnet, daß** der Neigungswinkel (α) durch einen Antrieb kontinuierlich veränderbar ist.

6. Vorrichtung zur Dreiphasentrennung nach zumindest einem der vorhergehenden Ansprüche, **dadurch gekennzeichnet, daß** die Außenfläche (2, 3) einen flexiblen Abschnitt (10, 11) aufweist.

7. Vorrichtung zur Dreiphasentrennung nach zumindest einem der vorhergehenden Ansprüche, **dadurch gekennzeichnet, daß** die Außenfläche (2, 3) eine gasdichte Folie oder ein Gewebe aufweist.

8. Vorrichtung zur Dreiphasentrennung nach zumindest einem der vorhergehenden Ansprüche, **dadurch gekennzeichnet, daß** der Gassammler (1, 15) ein für die Außenfläche (2, 3) bestimmtes Spannmittel (9, 18) aufweist.

9. Vorrichtung zur Dreiphasentrennung nach Anspruch 7, **dadurch gekennzeichnet, daß** das Spannmittel (9) ein Federelement ist.

10. Vorrichtung zur Dreiphasentrennung nach Anspruch 7, **dadurch gekennzeichnet, daß** das Spannmittel (18) einen Auftriebskörper (17) aufweist.

11. Vorrichtung zur Dreiphasentrennung nach Anspruch 10, **dadurch gekennzeichnet, daß** der Auftriebskörper (17) aus Bambus hergestellt ist.

12. Vorrichtung zur Dreiphasentrennung nach zumindest einem der vorhergehenden Ansprüche, **dadurch gekennzeichnet, daß** der Gassammler (1, 15) eine an mehreren Umlenkflächen (5, 6, 7, 8) gehaltene bewegliche Fläche aufweist, mit einem ersten, die Außenfläche (2, 3) bildenden Abschnitt (10, 11) und mit einem zweiten, zur Aufnahme des entstehenden Gases zurückgesetzten Abschnitt (15').

13. Vorrichtung zur Dreiphasentrennung nach Anspruch 9, **dadurch gekennzeichnet, daß** die flexible Fläche aus einem Schlauch (4) besteht.

14. Vorrichtung zur Dreiphasentrennung nach zumindest einem der vorhergehenden Ansprüche, **dadurch gekennzeichnet, daß** die Umlenkflächen (5, 6, 7, 8) an zumindest einer seitlichen formstabilen Aufnahme (Halteplatte 13) fixiert sind.

15. Vorrichtung zur Dreiphasentrennung nach zumindest einem der vorhergehenden Ansprüche, **dadurch gekennzeichnet, daß** die Vorrichtung mehrere zueinander benachbarte Gassammler (1, 15) aufweist, die einen zueinander einstellbaren Abstand aufweisen.

16. Vorrichtung zur Dreiphasentrennung nach zumindest einem der vorhergehenden Ansprüche, **dadurch gekennzeichnet, daß** die Außenfläche (2, 3) transmissiv ist.

17. Vorrichtung zur Dreiphasentrennung nach zumindest einem der vorhergehenden Ansprüche, **dadurch gekennzeichnet, daß** der Gassammler (1, 15) ein Verbindungsmittel (14) zur Positionierung bezüglich eines Druckgasspeichers der Vorrichtung zur Dreiphasentrennung aufweist.

## Claims

1. Apparatus for three-phase separation during treatment of waste water and sludge comprising a gas collector (1, 15) disposed in a settling basin for trapping gas which occurs during the conversion process in the apparatus for three-phase separation or is introduced therein, the gas collector (1, 15) having an outer face (2, 3) which is inclined relative to the horizontal and allows the sewage sludge to slide off, **characterised in that** the outer face (2, 3) has an angle of inclination (α) which may be adjusted for adaptation to different sewage sludges.

2. Apparatus for three-phase separation according to claim 1, **characterised in that** the gas collector (1, 15) has two outer faces (2, 3) inclined toward one another, the angles of inclination (α) of which can be adjusted independently from one another.

3. Apparatus for three-phase separation according to claims 1 or 2, **characterised in that** the angle of inclination (α) of the outer face (2, 3) simultaneously determines the respective volume (V) of the gas collector (1, 15) available for receiving gas.

4. Apparatus for three-phase separation according to any one of the preceding claims, **characterised in that** the angle of inclination (α) of a plurality of adjacent gas collectors (1, 15) is adjustable by a common drive.

5. Apparatus for three-phase separation according to any one of the preceding claims,
**characterised in that** the angle of inclination (α) is continuously variable by a drive.

6. Apparatus for three-phase separation according to any one of the preceding claims, **characterised in that** the outer face (2, 3) comprises a flexible portion (10, 11).

7. Apparatus for three-phase separation according to any one of the preceding claims, **characterised in that** the outer face (2, 3) comprises a gas-tight sheet material or a fabric.

8. Apparatus for three-phase separation according to any one of the preceding claims, **characterised in that** the gas collector (1, 15) comprises a tensioning device (9, 18) for the outer face (2, 3).

9. Apparatus for three-phase separation according to claim 7, **characterised in that** the tensioning device (9) is a spring element.

10. Apparatus for three-phase separation according to claim 7, **characterised in that** the tensioning device (18) comprises a float member (17).

11. Apparatus for three-phase separation according to claim 10, **characterised in that** the float member (17) is produced from bamboo.

12. Apparatus for three-phase separation according to any one of the preceding claims, **characterised in that** the gas collector (1, 15) comprises a movable face supported on a plurality of deflecting faces (5, 6, 7, 8), the movable face comprising a first portion (10, 11), which forms the outer face (2, 3), and a second, set-back portion (15') for receiving gas produced.

13. Apparatus for three-phase separation according to claim 9, **characterised in that** the flexible face is formed by a tube (4).

14. Apparatus for three-phase separation according to any one of the preceding claims, **characterised in that** the deflecting faces (5, 6, 7, 8) are fixed to at least one lateral, dimensionally stable receiver (retaining plate 13).

15. Apparatus for three-phase separation according to any one of the preceding claims, **characterised in that** the apparatus comprises a plurality of neighbouring gas collectors (1, 15) spaced apart an adjustable distance from one another.

16. Apparatus for three-phase separation according to any one of the preceding claims, **characterised in that** the outer face (2, 3) is transmissive.

17. Apparatus for three-phase separation according to any one of the preceding claims, **characterised in that** the gas collector (1, 15) comprises a connection means (14) for positioning with respect to a compressed gas accumulator of the apparatus for three-phase separation.

## Revendications

1. Dispositif pour la séparation en trois phases dans le traitement des eaux
résiduaires et de la boue, comprenant un collecteur de gaz (1, 15) disposé dans un bassin de décantation, pour collecter le gaz qui se dégage dans le processus de transformation dans le dispositif de séparation en trois phases, ou bien qui est introduit dans ce dispositif, le collecteur de gaz (1, 15) présentant une surface extérieure (2, 3) qui permet à la boue de clarification de glisser vers le bas et qui est inclinée sur l'horizontale, **caractérisé en ce que** la surface extérieure (2, 3) présente un angle d'inclinaison (α) qui peut être réglé pour l'adaptation à différentes boues de clarification.

2. Dispositif pour la séparation en trois phases selon la revendication 1,
**caractérisé en ce que** le collecteur de gaz (1, 15) présente deux surfaces extérieures (2, 3) inclinées l'une par rapport à l'autre qui peuvent être réglées en angle d'inclinaison (α) indépendamment l'une de l'autre.

3. Dispositif pour la séparation en trois phases selon les revendications 1 ou 2,
**caractérisé en ce que** le volume (V) du collecteur de gaz (1, 15) disponible à chaque fois pour collecter le gaz est lui-aussi déterminé en même temps par l'angle d'inclinaison réglé (α) de la surface extérieure (2, 3).

4. Dispositif pour la séparation en trois phases selon au moins une des
revendications précédentes, **caractérisé en ce que** plusieurs collecteurs de gaz (1, 15) adjacents peuvent être réglés en angle d'inclinaison (α) par un entraînement commun.

5. Dispositif pour la séparation en trois phases selon au moins une des
revendications précédentes, **caractérisé en ce que** l'angle d'inclinaison (α) peut être modifié par variation continue par un entraînement.

6. Dispositif pour la séparation en trois phases selon au moins une des
revendications précédentes, **caractérisé en ce que** la surface extérieure (2, 3) présente un segment flexible (10, 11).

7. Dispositif pour la séparation en trois phases selon au moins une des
revendications précédentes, **caractérisé en ce que** la surface extérieure (2, 3) comprend une feuille ou un tissu imperméable aux gaz.

8. Dispositif pour la séparation en trois phases selon au moins une des
revendications précédentes, **caractérisé en ce que** le collecteur de gaz (1, 15) présente un moyen de tension (9, 18) prévu pour la surface extérieure (2, 3).

9. Dispositif pour la séparation en trois phases selon la revendication 7,
**caractérisé en ce que** le moyen de tension (9) est un élément élastique.

10. Dispositif pour la séparation en trois phases selon la revendication 7,
**caractérisé en ce que** le moyen de tension (18) est un flotteur (17).

11. Dispositif pour la séparation en trois phases selon la revendication 10,
**caractérisé en ce que** le flotteur (17) est fabriqué en bambou.

12. Dispositif pour la séparation en trois phases selon au moins une des
revendications précédentes, **caractérisé en ce que** le collecteur de gaz (1, 15) comprend une surface mobile qui est montée sur plusieurs surfaces de renvoi (5, 6, 7, 8), et qui comprend un premier segment (10, 11) formant la surface extérieure (2, 3) et un deuxième segment (15') placé en retrait pour collecter le gaz dégagé.

13. Dispositif pour la séparation en trois phases selon la revendication 9,
**caractérisé en ce que** la surface flexible est constituée par un tuyau (4).

14. Dispositif pour la séparation en trois phases selon au moins une des
revendications précédentes, **caractérisé en ce que** les surfaces de renvoi (5, 6, 7, 8) sont fixées à un support latéral rigide (une plaque support 13).

15. Dispositif pour la séparation en trois phases selon au moins une des
revendications précédentes, **caractérisé en ce que** le dispositif présente plusieurs collecteurs de gaz (1, 15) adjacents entre eux qui présentent une distance d'écartement mutuel réglable.

16. Dispositif pour la séparation en trois phases selon au moins une des
revendications précédentes, **caractérisé en ce que** la surface extérieure (2, 3) est transmissive.

17. Dispositif pour la séparation en trois phases selon au moins une des
revendications précédentes, **caractérisé en ce que** le collecteur de gaz (1, 15) possède un moyen de liaison (14) servant à le positionner par rapport à un accumulateur de gaz comprimé du dispositif pour la séparation en trois phases.
